Europäisches Patentamt

(19) European Patent Office

Office européen des brevets

(11) Publication number : **0 669 138 A2**

(12) # EUROPEAN PATENT APPLICATION

(21) Application number : **95300884.4**

(51) Int. Cl.[6] : **A61L 31/00, A61L 27/00**

(22) Date of filing : **13.02.95**

(30) Priority : **24.02.94 US 201442**

(43) Date of publication of application :
**30.08.95 Bulletin 95/35**

(84) Designated Contracting States :
**AT BE CH DE DK ES FR GB GR IE IT LI LU MC NL PT SE**

(71) Applicant : **RESEARCH DEVELOPMENT FOUNDATION**
**402 North Division Street**
**Carson City Nevada 89703 (US)**

(72) Inventor : **Young, Ronald L.**
**843 Merridel Street**
**Houston, Texas 77024 (US)**

(74) Representative : **Wilkinson, Stephen John**
**Stevens, Hewlett & Perkins**
**1 St. Augustine's Place**
**Bristol BS1 4UD (GB)**

(54) **Amniotic membrane graft or wrap to prevent adhesions or bleeding of internal organs.**

(57)    Disclosed is a specially treated sheet of amnion, such as an amniotic membrane which prevents adhesions following injury, such as surgical injury and prevents bleeding of internal organs caused by injury. The amniotic sheet has its cellular monolayer removed exposing basement membrane, collagen types I, II, III, laminin, fibronectins, and other glycoprotein components which promote wound healing and remove antigenic sites in the cellular layer which may elicit an immune response. The amniotic material is sterilized and may be cross-linked. It is applied as a single layer to an injured surface subject to adhesions or wrapped on internal organs subject to bleeding, and can be sutured or applied by a liquid anti-adhesive adjuvant. The graft can be applied to a wet as well as a dry surface.

EP 0 669 138 A2

Field of the Invention

The present invention is in the field of preventing or reducing adhesions following injury, and particularly following surgical injury, and to preventing bleeding of internal organs following injury.

This application is a continuation-in-part of Application Serial No. 07/777,961 filed October 17, 1991, now abandoned

Background of the Invention

Adhesions may result from a great number of medical conditions or from surgical intervention. Illness leading to adhesion formation includes pelvic inflammatory or other pelvic or abdominal inflammatory processes resulting either from infection or endometriosis. The surgical procedures required by these and other pathologic conditions, e.g. cysts, tumors, etc., may also result in adhesion formation. Adhesions may, in turn, be associated with infertility by causing occlusion of the fallopian tubes or by interfering with tubal/ovarian function, inhibition of ovum pickup being the best example. It is postulated that the formation of adhesions evolves from trauma to serosal surfaces followed by release of a fibrinogen-rich exudate and preceding on to fibrin formation. This leads either to thick or filmy adhesive bands which may bridge the pelvic organs or tissues or to the dense fixation of these structures to each other. The presence of pelvic or abdominal adhesions is known to be a major cause of infertility in the human female.

Through the years a great number of natural and synthetic graft materials has been employed in an effort to reduce adhesion formation on traumatized surfaces, however, with only marginally successful results. Natural materials have included peritoneum, omentum, fat, and amnion, as well as amnion plus chorion. Synthetic materials including polyvinyl alcohol film and tantalum foil were used in the past, and, more recently, barriers consisting of Gelfilm and Gelfoam paste (Upjohn Co., Kalamazoo, Michigan), Surgicel (Johnson & Johnson, New Brunswick, New Jersey), and Silastic (Dow-Corning, Midland, Michigan), as well as meshes of Gore-Tex (Gore-Tex, Gore, Texas) and Interceed (Johnson & Johnson, New Brunswick, New Jersey) have been employed. The newer materials have led to more promising results.

Injury to internal organs, such as the liver, pancreas, spleen, kidney, and the like resulting in bleeding has been a problem over the years resulting in many cases in loss of the organ and death because of ineffective means and methods of preventing such bleeding.

The use of human amnion as a surgical adjunct has a long history. An excellent review has been published by Trelford and Trelford-Sauder. Amnion has been tried unsuccessfully to prevent pelvic and abdominal adhesions in a number of experimental animal models, as well as in human patients. The natural membrane has been tried in tubal surgery, and there exists an extensive experience with its use in vaginal reconstructive surgery in women. Other applications include repair of conjunctival defects, reconstruction of the bile duct, and prevention of meningocerebral adhesions following craniotomy. Its primary role in humans, however, has remained in the areas of burns, ulcers, and other skin trauma and in wound healing.

Prior to the present invention, no substantial literature existed describing the potential of human amnion in preventing intra-abdominal adhesions in humans, although some progress has been made in this area through the use of a number of synthetic agents. Badaway, et al. recently reported on the intra-abdominal application of amniotic membranes to prevent adhesions in the rat model. They noted little effect in inhibiting adhesion formation on serosal surfaces but observed somewhat better results on the parietal peritoneum. The explanations which they offered for the lack of success involved problems with post-operative organ immobility and blood pooling, both of which may play a role in adhesion formation after human surgery.

No substantial literature exists describing wrapping of internal organs with amniotic membranes to prevent bleeding following injury to injured internal organs such as the liver, pancreas, spleen, kidney, and the like.

A study of the literature reveals almost as many different methods of preparing and storing the membranes as there are case or experimental reports. Poor results were obtained with glutaraldehyde-treated membranes (unpublished data), as well as equally unsatisfactory experience elsewhere with alcohol pretreatment and oven drying or simple freezing in saline.

It would be highly advantageous to provide sheets of amniotic material, such as membranes, from human placenta which successfully prevent or reduce internal adhesions following injury, such as following surgical injury, and which promote healing and do not elicit an immune response.

It would be highly advantageous to provide wrapping or covering of injured bleeding internal organs with such amniotic sheets, such as membranes, which successfully stops bleeding.

Summary of the Invention

The present invention is directed to specially prepared amnion sheets or membranes, their process of making and use which successfully prevents or reduces adhesions due to injury, such as surgical injury or injuries to internal organs which successfully prevents bleeding, and which promotes healing and does not elicit an immune response.

In one aspect of the invention, a graft suitable for preventing or reducing adhesions on an internal organ of a patient is provided which comprises a sterilized sheet of amniotic material or membrane free of antigenic sites which avoids eliciting an immune response and has exposed basement membrane, collagen types I, II, III, laminin, fibronectins, and other glycoprotein components exposed which promote healing of the injured organ on contact.

The foregoing may be accomplished by removal of the cellular layer, preferably on the fetal side of the amniotic material from human placenta exposes basement membrane and collagen types I, II, III, laminin, fibronectins, and other glycoprotein components of the amniotic material; and contact with these components is believed to enhance wound healing. Also, removal of the cellular layer removes antigenic sites that may elicit an immune response. For example, removal of the cellular monolayer, preferably by trypsin washing, and then sterilizing, such as by gamma irradiation, according to the present invention is successful in preventing or reducing adhesions. The membranes thus prepared underwent adequate neovascularization and caused no significant inflammatory infiltration. This also supports a conclusion of no significant immunological reaction induced by the membranes, as also observed in the Badaway study. In the present invention, the cellular monolayer may be removed by any suitable method which completely removes it, for example by other proteases such as pepsin; however, ficin is not satisfactory since it is specific only for cysteine residues which occur less frequently in proteins and thus does not completely remove the cellular monolayer. Also, the amniotic membrane with its layer removed may be sterilized by other means, such as chemically with glutaraldehyde which also cross-links its collagen.

The thinness and the exceptional compliant quality of the membranes made them extremely facile to use. Single layer application of the membrane is mandatory in the pelvis and abdomen to prevent fibrosis formation within the membrane itself. Although the synthetic grafts are applied without suturing, these membranes can be fixed in place using microsutures or the use of liquid anti-adhesive adjuvants such as dextran. An added technical advantage to the use of these amniotic membranes as opposed to the synthetic meshes is the fact that they can be applied and sutured over surfaces not perfectly dry.

Accordingly, it is an object of the present invention to provide a graft or a wrap of sterilized amniotic material free of antigenic sites thereby avoiding eliciting an immune response and having basement membrane, collagen types I, II, III, laminin, fibronectins, and other glycoprotein components exposed to promote wound healing on contact.

Another object of the present invention to provide a graft or wrap of sterilized amniotic membrane having its cellular layer removed exposing collagen, other glycoproteins, and basement membrane to promote wound healing and to remove antigenic sites in the cellular layer which may elicit an immune response.

It is a further object of the present invention to provide a sterilized graft or wrap of amniotic membrane from human placenta having its cellular layer removed by proteolytic treatment for preventing adhesions in a patient following injury or bleeding of internal organs.

It is a further object of the present invention to provide such a graft or wrap in which the cellular monolayer on the fetal side of the amniotic membrane is removed by trypsin.

It is a further object of the present invention to provide such a graft or wrap in which the amniotic membrane having its cellular monolayer material removed is sterilized and has its collagen cross-linked by gamma irradiation.

It is a further object of the present invention to provide a method of preparing a graft or wrap for preventing adhesions or bleeding of internal organs in a patient due to injury by removing cellular material on the fetal side of the amniotic membrane then sterilizing the membrane.

It is still a further object of the present invention to provide a method of preventing adhesions or bleeding of internal organs following injury in a patient, comprising applying a graft of amniotic material according to the invention to the surface of a patient subject to the formation of adhesion or bleeding.

Other and further objects, features, and advantages appear throughout the specification and claims.

Description of Preferred Embodiments

The graft of the invention comprises a single layer of human amniotic material, such as a membrane, free of antigenic sites which avoids an immune response and having it exposed basement membrane, collagen types I, II, III, laminin, fibronectins, and other glycoprotein components which promote healing of an injured organ on contact. Preferably, this is accomplished by having the cellular layer removed from the membrane exposing its collagen and basement membrane components which enhances wound healing and removes antigenic sites that may elicit an immune response. Preferably, the cellular material is removed by a protease effective to remove it, preferably with trypsin which works on multiple sites on protein (lysine and arginine), and almost all proteins are susceptible to trypsin proteolysis. Ficin on the other hand is specific for cysteine residues which occur less frequently in proteins. Other proteases are effective to remove the cellular layer, such as chynotripsin and pepsin. Such a human single layer amniotic membrane successfully prevents or reduces adhesions and prevents bleeding following injury, such as surgical injury. Excellent results have been obtained by employing these treated single layered grafts.

The proposed method of preparing these amniotic membrane grafts according to the invention comprises harvesting freshly delivered human placentas, preferably taken at the time of cesarean section, manually separating the amniotic membranes from the chorion, washing in distilled water, removing the cellular material from the fetal side of the amniotic membranes, such as by soaking them in a solution of trypsin, and then sterilizing the collagen of the amniotic membrane such as by gamma irradiation or by chemicals such as glutaraldehyde.

The method of preventing adhesions and bleeding of internal organs according to the invention comprises applying a single layer sheet of the graft to the injured surface with or without suturing or by use of liquid anti-adhesive adjuvants such as dextran. Advantageously, these amniotic membrane grafts and wraps can be applied and sutured over surfaces not perfectly dry.

## EXAMPLE 1

### Materials and Methods

The amniotic membranes were harvested from freshly delivered human placentas taken at the time of cesarean section. The amniotic membranes were manually separated from the chorion and washed in distilled water. The clean membranes were first treated by soaking for three hours in a 10% solution of trypsin. Subsequently, they were irradiated with gamma irradiation to sterilize them in the following manner. First, the entire membrane underwent an 8 hour 20 minute irradiation at 60,000 rads per hour for a total dose of 500,000 rads. The membranes were then cut into smaller squares of approximately 2 x 2 cm and reradiated at 60,000 rads per hour for a total of 33 hours 20 minutes equivalent to a 2 million rad dose. The small squares thus prepared were frozen at -70°C in distilled water to maintain them until they were used (within 4 weeks). Just prior to use, the membranes were thawed at 22°C. No antibiotics were used during this process.

### Surgical Procedure

Study animals consisted of multiparous female New Zealand rabbits each weighing at least 3.5 kilograms to ensure adequate size of the pelvic organs. Six rabbits were assigned to each group A through C, and 18 to group D. At the time of surgery the rabbits were anesthetized with a mixture consisting of ketamine, promazine, and xylozine at a dosage of 1 cc/kg of body weight. The abdomen was shaved, subjected to sterile preparation and draped. Sterile microsurgical techniques under the operating microscope were employed as previously described by Badaway, et al. Experimental injuries consisted of a series of incisions through the serosal and muscularis layers of the uterine horn extending into the endometrial cavity with frequent avulsion of the mucosa. The cuts, 1 cm long and spaced 5 millimeters apart, were created with microscissors. Membrane grafts approximately 1 x 2 cm in size were sutured into place over the lesion in a single layer using multiple interrupted sutures of 7-0 maxon. The maternal side of the membrane was placed against the injury, and the fetal side faced into the abdominal cavity. Following surgery, the abdomen was closed in three layers and a sterile dressing left in place for 72 hours. All animals received procaine penicillin at a dosage of 50 ml per kg IM qOD x 5 doses postoperatively and were maintained in a vivarium at 27°C with 40-70% humidity and given Purina Rabbit Chow pellets and water ad libitum.

### Description of Experimental Groups

Animals were randomly assigned to 3 groups of 6 animals each and a fourth group of 18 animals. Each rabbit was subjected to 2 surgical procedures, the initial laparotomy including the designated operative procedure, and a second look laparotomy to evaluate the effects of the experimental intervention. The groups were as follows: Group A (n + 6) was the background control group. The abdomen in this group was opened, exposed to no specific injury or treatment and closed. Group B (n = 6) was the model control group in which controlled injuries, as described above, were made on one uterine horn of each animal. The contralateral horn was not injured, and no therapeutic interventions were made on either horn. Group C (n = 6) was the first treatment group where injuries were carried out on one uterine horn as in Group B, and then both injured and non-injured horns were covered with membrane grafts held in place with microsutures. Group D (n=16) formed the second treatment group in which both uterine horns were experimentally injured in a similar manner. One horn was then treated by suturing a membrane into place, and the contralateral horn was treated with interrupted, hemostatic microsutures of 7-0 maxon.

Thirty days after the initial laparotomy and surgical intervention each animal was reoperated, and adhesions were photographed and evaluated with regard to their presence or absence, percentage of surface included, and graded as to adhesion quality (thin, thick, filmy, dense). Statistical analysis was performed using Fisher's Exact Test. It was considered significant at $p < 0.05$.

Results

All results are summarized in Table 1.

Table 1   Comparison of Results in Membrane-Treated
Versus Untreated Uterine Hours Following Experimental Injury

| Group | Horn | Surgical Procedure | Number with Adhesions (%) | Types of Ahesions | Percentage of Surface Affected |
|---|---|---|---|---|---|
| A | R | none | 0 | | |
| n = 6 | L | none | 0 | | |
| B | R | Incisions | 6 (100) a | dense, thick | 75 |
| n = 6 | L | none | 0 | | |
| C | R | Incisions + membrane | 0 | | |
| n = 6 | L | Membrane only | 1 (17) | filmy, thin | 10 |
| D | R | Incisions + membrane | 3 (17) a | filmy, thin | 10 |
| n = 18 | L | Insicions + sutures | 18 (100) | dense, thick | 80 |

a   Right vs left horn.  Fisher's Exact Test.  In each case $p < 0.05$.

The background control group plus model control Group B confirmed the validity of the model by demonstrating that there were no background adhesions from laparotomy alone and that the experimental injury was sufficient to cause dense adhesions in 100% of the cases if untreated. These included surface adhesions as well as loop-to-loop adhesions leading to severe tortuosity of the involved horn. It was further noted that there was no crossover of these adhesions to the injured contralateral horn (p = 0.002).

Experimental Group C showed no significant difference (p = 0.5) in adhesion formation on sites of membrane grafts placed over injured versus non-injured uterine horns. Thin, filmy adhesions were found in only one case on the non-injured horn and in no cases on the injured side. Finally, in experimental Group D, membrane grafts significantly reduced the formation of adhesions as compared to those found at the site of hemostatic microsutures. Dense, thick adhesions over an average of 80% of the surface area of the sites of injury were noted on the horns treated with the sutures in 100% of the cases. In contrast, only 17% of the injured horns (p = 0.0000003) which had been covered with membrane grafts showed any adhesions, and these were thin, filmy, and covered only about 10% of the injured/grafted area. Histological studies showed that the membranes were integrated with the serosal layer and showed neovascularization at the site of the graft. Minimal polymorphonuclear infiltration of the serosal surfaces was present suggesting no significant immunological response.

The thinness and the exceptional compliant quality of the single layer membranes made them extremely facile to use. Single layer application is mandatory in the pelvis and abdomen to prevent fibrosis formation within the membrane itself. Also, the synthetic grafts are applied without suturing, and in the foregoing examples the membranes were fixed in place using microsutures. In the human, this adds the potential for concomitant use of liquid anti-adhesive adjuvants such as dextran. Also, an added technical advantage to the use of these amniotic membranes, as opposed apparently to the synthetic meshes is the fact that they can be applied and- sutured over surfaces not perfectly dry.

EXAMPLE 2

In this example, formation of adhesions involving the parietal peritoneum was prevented by these membranes as prepared and as used in Example 1 which improves the outcome of procedures involving extensive endometriosis or pelvic sidewall adhesions of the adnexal structures.

EXAMPLE 3

In this example, the treatment of the amniotic membranes was the same as that in Example 1 except that chynotripsin and pepsin were each substituted for trypsin with satisfactory results.

EXAMPLE 4

In this example, the treatment of the amniotic membranes is the same as that in Examples 1 and 3 except that the sterilization of the amniotic membrane after removal of its cellular layer was done by treatment with glutaraldehyde with satisfactory results.

EXAMPLE 5

In this example, sheets of the amniotic membranes are wrapped about or cover injured and bleeding portions of internal organs, the liver, pancreas, spleen, kidney preferably with the exposed components of the amnion against the injured portions and successfully prevented such bleeding. The amniotic membranes are applied into place as in the preceding examples. As previously mentioned, these amniotic membrane grafts and wraps can be applied and cover wet surfaces, as well as dry ones.

The foregoing animal studies are excellent models for prevention or reduction of adhesions and bleeding of internal organs in human beings. The term "patient" as used herein includes human and animal patients.

The present invention, therefore, is well suited and adapted to attain the objects and ends and has the features and advantages mentioned as well as others inherent therein.

While presently preferred embodiments have been given for the purpose of disclosure, changes can be made therein which are within the spirit of the invention as defined by the scope of the appended claims.

**Claims**

1. A graft suitable for reducing adhesions on an injured internal organ of a patient comprising,

    a sterilized sheet of amniotic material from human placenta free of antigenic sites and having exposed basement membrane and collagen types I, II, III, laminin, fibronectins, and other glycoprotein components of the amniotic material to promote healing of the injured organ on contact.

2. The graft of Claim 1 where,

    the sheet of amniotic material has its cellular layer removed thereby removing the antigenic sites and exposing the components of the amniotic material.

3. The graft of Claim 2 where,
   the cellular layer has been removed by treatment with trypsin.

4. The graft of Claim 1 where,
   the collagen is cross-linked.

5. The graft of Claim 2 where,
   the collagen is cross-linked.

6. The graft of Claim 3 where,
   the collagen is cross-linked.

7. The graft of Claim 1 where,
   the collagen is cross-linked by gamma irradiation of from 0.25M to 2.5M rads.

8. The graft of Claim 2 where,
   the collagen is cross-linked by gamma irradiation of from 0.25M to 2.5M rads.

9. The graft of Claim 1 where,
   the sheet of amniotic material is a single sheet.

10. The graft of Claim 2 where,
    the sheet of amniotic material is a single sheet.

11. The graft of Claim 3 where,
    the sheet of amniotic material is a single sheet.

12. The graft of Claim 4 where,
    the sheet of amniotic material is a single sheet.

13. The graft of Claim 5 where,
    the sheet of amniotic material is a single sheet.

14. The graft of Claim 6 where,
    the sheet of amniotic material is a single sheet.

15. A method of preparing a graft suitable for reducing adhesions to an injured internal organ comprising,
    removing cellular material from an amniotic membrane from human placenta thereby exposing basement membrane, collagen types I, II, III, laminin, fibronectins, and other glycoprotein components of the amniotic material to promote healing when applied to the injured organ, and thereby removing antigenic sites in the cellular material, and
    sterilizing the amniotic membrane.

16. The method of Claim 15 where,
    the cellular material is removed by treatment with trypsin.

17. The method of Claim 15 including,
    cross-linking the amniotic material.

18. The method of Claim 16 including,
    cross-linking the amniotic material.

19. The method of Claim 16 including,
    sterilizing and cross-linking the amniotic material by gamma irradiation of from 0.25M to 2.5M rads.